# EUROPÄISCHE PATENTANMELDUNG

(11) **EP 2 540 342 A1**
(43) Veröffentlichungstag der Anmeldung: **02.01.2013**
(21) Anmeldenummer: 12167252.1
(22) Anmeldetag: 09.05.2012
(51) Int. Cl.: A61N 1/365, A61N 1/362

(54) **Herzstimulator zur Abgabe einer kardialen Kontraktilitätsmodulationstherapie**

(30) Priorität: 28.06.2011 US 201161501766 P
(71) Anmelder: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Lindner-Vogt, Karin L.

(57) **Zusammenfassung**

Die Erfindung betrifft einen Herzstimulator mit folgenden Bestandteilen:
- wenigstens einer Stimulationseinheit, die mit einer oder mehreren Stimulationselektroden verbunden oder zu verbinden und ausgebildet ist, zumindest unterschwellige Stimulationsimpulse für eine kardiale Kontraktionsmodulationstherapie abzugeben,
- einer Impedanzerfassungseinheit, die mit einer oder mehreren Elektroden verbunden oder zu verbinden und ausgebildet ist, eine infolge eines jeweiligen unterschwelligen Stimulationsimpulses auftretende Spannung oder Stromstärke zu erfassen und daraus einen jeweiligen Impedanzwert abzuleiten,
- einer Impedanzauswertungseinheit, die ausgebildet ist, wenigstens einen Herzkammervolumen-bezogenen Wert und/oder einen Atemzeitvolumen-bezogenen Wert zu bestimmen und
- einer Steuereinheit, die mit der Stimulationseinheit und der Herzrhythmuserfassungseinheit verbunden und ausgebildet ist, eine Stimulationsimpulsabgabe über die Stimulationseinheit derart zu steuern, dass der Herzstimulator unterschwellige Stimulationsimpulse für eine kardiale Kontraktionsmodulationstherapie abgeben kann.

Die Steuereinheit ist erfindungsgemäß mit der Impedanzauswertungseinheit verbunden und ausgebildet, eine Abgabe unterschwelliger Stimulationsimpulse für eine kardiale Kontraktionsmodulationstherapie in Abhängigkeit des Herzkammervolumen-bezogenen Wertes und/oder des Atemzeitvolumen-bezogenen Wertes zu steuern.

## Beschreibung

Die Erfindung betrifft einen Herzstimulator zur Abgabe einer kardialen Kontraktilitätsmodulationstherapie.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- und optional zusätzliche Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myocard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myocards. Während der Phase der Depolarisation sind die Herzmuskelzellen für eine Erregung unempfindlich, d.h. refraktär. Diese Zeit wird als Refraktärzeit bezeichnet. Die mit der Depolarisation und Repolarisation einhergehenden elektrischen Potentiale können wahrgenommen und deren zeitlicher Verlauf - als Elektrokardiogramm bezeichnet - ausgewertet werden.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender, natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlichen (intrinsischen) Ereignisse erfolgt durch Ableiten der elektrischen Potenziale des Myokards der jeweiligen Herzkammer mit Hilfe von Sensingelektroden, die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischerweise ist für das Sensing - d.h. die Wahrnehmung intrinsischer Ereignisse - ein Sensingelektrodenpaar vorgesehen, das von zwei benachbarten Elektroden, nämlich einer Spitzenelektrode (Tip-Elektrode) und einer Ringelektrode gebildet ist, von denen die Spitzenelektrode auch als Stimulationselektrode dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Sensingelektrode aufweisen kann.

Die Sensingelektroden sind im Betrieb des Herzstimulators mit entsprechenden Sensingeinheiten verbunden, die ausgebildet sind, ein jeweiliges über eine Sensingelektrode (bzw. ein Sensingelektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkte der Abgabe eines jeweiligen Stimulationsimpulses in Abhängigkeit einer jeweiligen Stimulationsrate geplant, die dem physiologischen Bedarf eines Patienten entsprechen soll, also bei größerer Anstrengung beispielsweise höher ist. Hierzu kann ein Herzstimulator mit einem oder mehreren Aktivitätssensoren ausgestattet sein, der beispielsweise ein nachfolgend näher beschriebener CLS-Sensor sein kann.

Die natürliche Einwirkung des autonomen Nervensystems auf die Herzrate (Herzfrequenz), die durch einen ratenadaptiven Herzstimulator nachgebildet werden, wird als Chronotropie bezeichnet.

Neben der Chronotropie wird die Herzleistung auch von dessen Kontraktilität bestimmt, deren Beeinflussung als Inotropie bezeichnet wird.

Zur Bestimmung der Kontraktilität eines Herzen ist es bekannt, in einem Gehäuse eines Herzstimulators (z.B. eines implantierbaren Herzschrittmachers) eine Impedanz- oder Leitfähigkeitsmesseinheit anzuordnen, die ausgebildet ist, ein uni- oder bipolares Impedanz-oder Leitfähigkeitsverlaufssignal zu erzeugen. Dazu werden während wenigstens eines Herzzyklus' mehrere Impedanz- oder Leitfähigkeitswerte oder ein entsprechender Impedanz- oder Leitfähigkeitsverlauf gemessen. Dies geschieht entweder unipolar durch Messen zwischen einer Neutralelektrode und einer Messelektrode oder zwischen zwei Messelektroden. Außerdem ist in dem Gehäuse eine Auswerteeinheit angeordnet, die zum Auswerten des Impedanz- oder Leitfähigkeitsverlaufs und zum Ableiten eines Kontraktilitätswertes aus dem Impedanz- oder Leitfähigkeitsverlauf dient. Elektrotherapiegeräte, die die Kontraktilität eines Herzens ermitteln können, bieten die Möglichkeit, einer von dem E-lektrotherapiegerät abzugebende Therapie an den jeweiligen Kontraktilitätszustand des Herzens des Patienten anzupassen.

Wie angedeutet, beschreibt die Kontraktilität einen inotropen Zustand eines Herzens. Die Kontraktilität beeinflusst die Kraft und die Geschwindigkeit einer myokardialen Kontraktion. Die Kontraktilität wird durch drei Mechanismen gesteuert:
eine direkte Steuerung durch das autonome Nervensystem (ANS),
den sogenannten Starling-Mechanismus, und
den sogenannten Bowditch-Effekt (Kraft-Frequenzkopplung).

Der Hauptmechanismus, die Steuerung der Kreislaufregulation durch das autonome Nervensystem, vergrößert die Kontraktilität und die Herzrate, wenn ein erhöhter metabolischer Bedarf vorliegt, beispielsweise bei körperlicher oder physischer Anstrengung, um eine geeignete Blutversorgung sicherzustellen. Beim gesunden Menschen bewirkt die Inotropie des Herzens somit einen Anstieg der Kontraktilität bei infolge erhöhten physiologischen Bedarfs.

Bei Patienten mit chronischem Herzversagen (Chronic Heart Failure, HF) nimmt die myokardiale Kontraktilität bis auf ein niedriges Niveau ab und die interventrikuläre Synchronisation wird verschlechtert. Dies wird von einem geringen Auswurfanteil (Ejection Fraction, EF) begleitet, sowie von einer geringen Lebensqualität und einer hohen Sterblichkeit. HF kommt in der Bevölkerung häufig vor. In jüngerer Zeit werden HF-Patienten mit Resynchronisationstherapie-Geräten, beispielsweise 3-Kammer-Herzschrittmachern oder Defibrillatoren behandelt. Ziel einer solchen Schrittmachertherapie ist es, die zwei Ventrikel eines Herzens durch biventrikuläre Stimulation zu synchronisieren, um das Zeitverhalten der Herzkammern zu verbessern und damit die Herzleistung. Eine derartige Therapie wird auch als kardiale Resynchronisationstherapie (Cardiac Resynchronisation Therapy, CRT) bezeichnet. Die kardiale Resynchronisationstherapie (CRT) ist hinreichend bekannt und wird z.B. von den Biotronik CRT-D Implantaten (Lumax HF-T) angeboten.

Da die Kontraktilität des Herzens physiologisch vom autonomen Nervensystem gesteuert wird, kann die die Erfassung der Kontraktilität auch zum Einstellen einer physiologisch adäquaten Stimulationsrate bei ratenadaptiven Herzschrittmachern genutzt werden. Diese weiter vorne bereits angesprochene Art der Stimulationsratensteuerung ist auch als Closed Loop Stimulation (CLS) bekannt.

Für die CLS wird der intrakardiale Impedanzverlauf nach Beginn der Ventrikelkontraktion gemessen. Diese Messung erfolgt sowohl bei intrinsischen als auch bei stimulierten Ereignissen. Es besteht eine direkte Abhängigkeit zwischen dem rechtsventrikulären Impedanzverlauf und der Kontraktionsdynamik. Die Kontraktionsdynamik wiederum ist ein Parameter für die Stimulation des Herzens durch den Sympathikus.

Die Closed Loop Stimulation dient wie gesagt der Stimulationsratensteuerung bei einem ratenadaptiven Herzschrittmacher.

Zur Steigerung der Kontraktilität einer Herzkammer ist die sogenannte kardiale Kontraktilitätsmodulationstherapie (Cardiac Contractility Modulation: CCM) bekannt.

Hierfür bietet beispielsweise die Firma Impulse Dynamics ein sogenanntes OPTIMIZER-System zur CCM-Therapie an. Dieses System umfasst einen Stimulationsimpulsgenerator, verbunden mit 3 Elektroden, von denen im Betrieb eine im Atrium und 2 am Septum des rechten Ventrikels eines Patienten angeordnet sind. Das Therapieprinzip beruht auf einer Abgabe von biphasischen Stimulationspulsen mit einer Amplitude von 7-10V und einer Gesamtimpulsdauer von ~20ms in die absolute Refraktärzeit des Ventrikels mit dem Ziel, die Kontraktilität zu steigern. Die Therapie wird dabei für bestimmte Zeiteinheiten des Tages (z.B. abwechselnd jeweils 1h ein, 1h aus) abgegeben.

Das Prinzip der kardialen Kontraktilitätsmodulationstherapie ist unter anderem auch in US 6,317,631 B1 beschrieben.

Die Wirkung der CCM-Therapie beruht - so wird derzeit vermutet - in einer Modifikation des zellulären Kalziumionenaustauschs und führt so zu einer gesteigerten Kontraktionskraft, die auch bei einem vorliegenden Vorhofflimmern einen therapeutischen Benefit zur Folge haben sollte. Dies ist zwar klinisch noch nicht nachgewiesen, ist pathophysiologisch aber nachvollziehbar.

US 2010/0069977 A1, US 2010/0069980 A1, US 2010/0069984 A1, US 2010/0069985 A1 beschreiben verschiedene Methoden, um eine CCM-Stimulation bedarfsgerecht abzugeben. Hier wird der Einsatz von physiologischen Sensoren, Nieren- oder Herzfunktionssensoren, Elektrolytsensoren, Serumsensoren (z.B. Kreatinin), Neurosensoren (Vagus, Sympathikus), Adverse Event Detektoren, Worsening Heart Failure Sensoren, MRT-Sensoren, Aktivitätssensoren, Schlafapnoe-Sensoren, Ischemiesensoren, Sensoren für den metabolischen Bedarf und Infarktsensoren sowie Herzrhythmus-abhängigen CCM-Steuerungen allgemein beschrieben.

US 2010/0069977 A1, US 2010/0069980 A1, US 2010/0069984 A1, US 2010/0069985 A1 beschreiben nicht die konkreten Implementierungsmethoden für die Ankopplung der CCM-Therapie an die benötigten Sensoren. Ebenso wird auch hier nicht auf die konkrete Ankopplung einer bedarfsgerechten CCM-Therapie an die Sensorsteuerungen von Schrittmacher- und ICD-Systemen eingegangen. Fast alle dort genannten Sensoren müssen zunächst neu entwickelt und für den Einsatz mit der CCM-Therapie verifiziert werden. Die meisten der zusätzlichen Sensoren stellen dabei komplexe Anforderungen an das Elektroden- bzw. Sensordesign.

Der Erfindung liegt die Aufgabe zugrunde, die kardiale Kontraktilitätsmodulationstherapie (Cardiac Contractility Modulation: CCM) bedarfsgerecht einzusetzen, d.h. derart zu steuern, dass diese Therapie immer nur dann aktiviert wird, wenn ein tatsächlicher Therapiebedarf besteht.

Erfindungsgemäß wird diese Aufgabe durch einen Herzstimulator mit folgenden Bestandteilen gelöst:
- wenigstens einer Stimulationseinheit, die mit einer oder mehreren Stimulationselektroden verbunden oder zu verbinden und ausgebildet ist, zumindest unterschwellige Stimulationsimpulse für eine kardiale Kontraktionsmodulationstherapie abzugeben,
- einer Impedanzerfassungseinheit, die mit einer oder mehreren Elektroden verbunden oder zu verbinden und ausgebildet ist, eine infolge eines jeweiligen unterschwelligen Stimulationsimpulses auftretende Spannung oder Stromstärke zu erfassen und daraus einen jeweiligen Impedanzwert abzuleiten,
- einer Impedanzauswertungseinheit, die ausgebildet ist, wenigstens einen Herzkammervolumen-bezogenen Wert und/oder einen Atemzeitvolumen-bezogenen Wert und/oder einen Kontraktionszeit-bezogenen Wert zu bestimmen und
- einer Steuereinheit, die mit der Stimulationseinheit und der Herzrhythmuserfassungseinheit verbunden und ausgebildet ist, eine Stimulationsimpulsabgabe über die Stimulationseinheit derart zu steuern, dass der Herzstimulator unterschwellige Stimulationsimpulse für eine kardiale Kontraktionsmodulationstherapie abgeben kann.

Die Steuereinheit ist erfindungsgemäß mit der Impedanzauswertungseinheit verbunden und ausgebildet, eine Abgabe unterschwelliger Stimulationsimpulse für eine kardiale Kontraktionsmodulationstherapie in Abhängigkeit des Herzkammervolumen-bezogenen Wertes und/oder des Atemzeitvolumen-bezogenen Wertes und/oder des Kontraktionszeit-bezogenen Wertes zu steuern.

Die Impedanzauswertungseinheit ist beispielsweise Teil eines CLS-Sensors oder HDS-Sensors zum Generieren eines Steuersignals für eine Stimulationsratensteuerung auf Basis Herzkammervolumen-basierter bzw. Kontraktionszeit-basierter Werte. An Stelle eines CLS-Sensors kann auch ein Atemminutenvolumensensor zum Einsatz kommen, der wenigstens zum Teil von der Impedanzauswertungseinheit gebildet wird. Alternativ kommt als Sensor auch eine Kombination eines CLS- und Atemminutenvolumensensors in Frage, der ebenfalls wenigstens eine Impedanzauswertungseinheit aufweist.

Die Erfindung schließt die Erkenntnis ein, dass die vorne beschriebene CCM-Therapie einer V00-Stimulation entspricht, d.h. die Stimulation wird ohne eine Bedarfsregelung abgegeben. Um den Stromverbrauch (und eventuelle negative Effekte) zu reduzieren, wird diese sehr energieintensive Therapie bisher immer nur für einige Stunden des Tages aktiviert.

Das die bislang vorliegenden klinischen Daten eine tatsächliche Kontraktilitätssteigerung dieser CCM-Therapie gezeigt haben *[siehe* Borggrefe et al. "Randomized, double blind study of non-excitatory, cardiac contractility modulation electrical impulses for symptomatic heart failure. " Eur Heart J 2008 29: 1019-1028*]*, lässt sich vermuten, dass diese Therapie tatsächlich als Option für sog. CRT-Non-Responder erfolgreich eingesetzt werden kann.

Ein weiterer potentieller Nachteil der ungesteuerten Therapie besteht in der unkontrollierten Kontraktilitätssteigerung durch die CCM-Stimulation. Die Kontraktilität wird hier auch dann gesteigert, wenn der metabolische Bedarf des Körpers keine gesteigerte Kontraktilität erfordert. Dies bedeutet gleichzeitig das Risiko, dass durch diese unspezifische Therapie die Herzinsuffizienz auch weiter verschlechterte werden kann, das durch die Kontraktilitätssteigerung der metabolische Bedarf des Herzens dauerhaft erhöht wird. Kann dieser nicht immer bedient werden, kommt es möglicherweise zu einer progredienten Dekompensation anstelle des erhofften Revers-Remodeling des Myokards.

Der erfindungsgemäße Herzstimulator kann beispielsweise ein CLS-gesteuerter Stimulator (Schrittmacher oder ICD/CRT-D) sein, der um eine bedarfsgesteuerte CCM-Therapieeinheit derart erweitert ist, dass die CCM-Therapie immer nur dann abgegeben wird, wenn der kardiale Zustand dies tatsächlich auch erfordert. Damit kann der bislang übermäßige CCM-Stromverbrauch signifikant gesenkt und eine mögliche fortschreitende Dekompensation vermieden werden.

In einer bevorzugten Ausführungsvariante weist der Herzstimulator eine Herzrhythmuserfassungseinheit auf, die ausgebildet ist eine intrinsische Herzfrequenz zu erfassen, wobei die Steuereinheit mit der Herzrhythmus-Erfassungseinheit verbunden und ausgebildet ist, aus dem Herzkammervolumen-bezogenen Wert eine indizierte Herzfrequenz abzuleiten und eine Abgabe unterschwelliger Stimulationsimpulse für eine kardiale Kontraktionsmodulationstherapie zu unterbinden, wenn die durch den Herzkammervolumen-bezogenen Wert indizierte Herzfrequenz oder eine Änderung der indizierten Herzfrequenz einer intrinsischen Herzfrequenz bzw. der Änderung einer intrinsischen Herzfrequenz qualitativ entspricht. Ein solcher Herzstimulator nutzt die Kenntnis, dass ein erhöhter metabolischer Bedarf beim gesunden Menschen sowohl einer Steigerung der Herzrate (Herzfrequenz) als auch eine damit im Einklang stehende Steigerung der Kontraktilität bewirkt, so dass aus einer natürlichen Steigerung der Herzrate, also einer Steigerung der Sinusrhythmus auf eine adäquate Steigerung der Kontraktilität geschlossen werden kann. Zeigt sich diese nach Auswertung er Herzkammervolumen-bezogenen Werte nicht, kann eine kardiale Kontraktionsmodulationstherapie - also die Abgabe entsprechender Stimulationsimpulse während der absoluten Refraktärzeit eines jeweiligen Herzen - ausgelöst werden.

Es ergeben sich folgende alternative Untervarianten:
Eine kardiale Kontraktionsmodulationstherapie wird immer dann einleitet, wenn der CLS-Sensor eine Herzfrequenzsteigerung vorgibt (Mitkopplung).
Eine kardiale Kontraktionsmodulationstherapie wird immer dann einleitet, wenn der CLS-Sensor eine Reduktion der Herzfrequenz vorgibt (Gegenkopplung).

Besonders bevorzugt ist eine Ausführungsvariante, bei der die Steuereinheit ausgebildet ist, eine kardiale Kontraktionsmodulationstherapie immer dann zu starten, wenn die intrinsische Herzfrequenz des Patienten, bei nachgewiesenem Sinusrhythmus ansteigt, der vom CLS-Sensor vorgegebene Frequenzanstieg jedoch hinter dem intrinsischen Herzfrequenzanstieg zurückbleibt (entspricht einem diagnostizierten Kontraktilitätsdefizit bei gesteigertem metabolischem Bedarf).

Gemäß weiterer bevorzugter Ausführungsvarianten ist neben der Impedanzauswertungseinheit ein (weitere) Aktivitätssensor, z.B. ein an sich bekanntes Akzelerometer vorgesehen, der ebenfalls ein Ausgangssignal liefert, dass einen Hinweis auf den metabolischen Bedarf eines Patienten gibt. Das Ausgangssignal des Akzelerometers kann dann von der Steuereinheit zur Plausibilitätskontrolle verarbeitet werden.

Vorzugsweise ist die Steuereinheit ausgebildet, eine kardiale Kontraktionsmodulationstherapie immer dann einzuleiten, wenn das Ausgangssignal der Impedanzauswertungseinheit eine Herzfrequenzsteigerung indiziert, das Akzelerometersignal hingegen keine Frequenzsteigerung anzeigt.

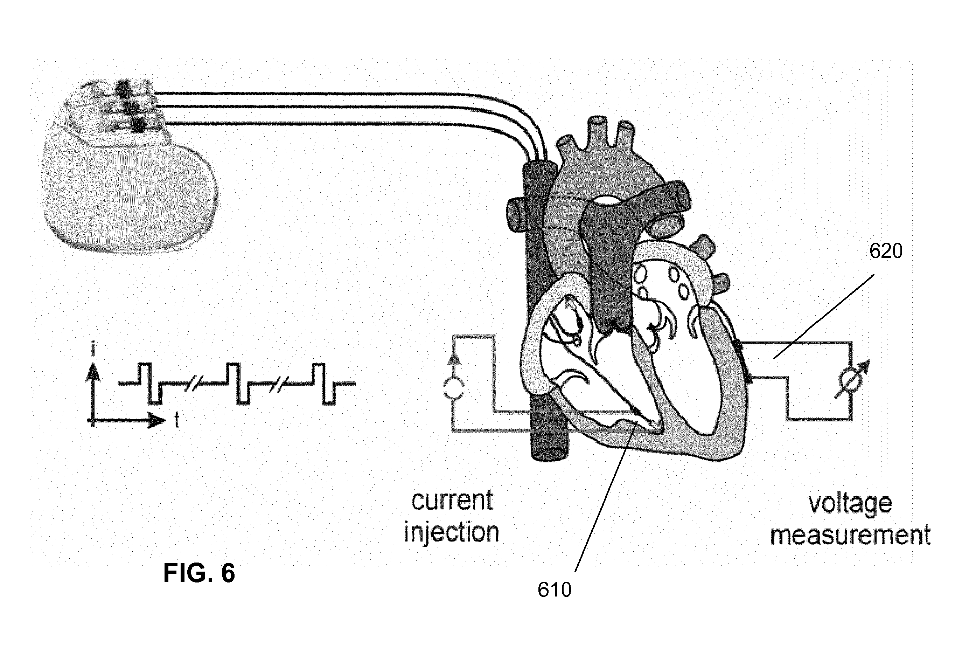

Gemäß einer weiteren, bevorzugten Ausführungsvariante ist ein jeweiliger für eine Impedanzmessung genutzter unterschwelliger Strom- oder Spannungsimpulse ein Stimulationsimpuls für die Kontraktionsmodulationstherapie. Ein solcher Herzstimulator erlaubt den Einsatz eines kombinierten CLS- und HDS-Sensors zur Steuerung der kardialen Kontraktilitätsmodulationstherapie (CCM), wobei der HDS-Sensor sich der ohnehin vorhandenen CCM-Konstantstrompulse zur haemodynamischen Sensorik, d.h. zur Bestimmung Herzkammervolumen-bezogener Werte bedient. er bevorzugte Herzstimulator nutzt die Tatsache, dass die für die CCM-Therapie bevorzugten bipolaren Konstantstrompulse prinzipiell den im sog. HDS-Sensorprinzip genutzten Strompulsen entsprechen. Jedoch haben die CCM-Strompulse ein Vielfaches der Stromstärke, so dass hier ein erheblich besseres Signal-Rausch-Verhältnis zu erwarten ist und damit einen einfachere Sensorauswertung möglich ist. Allerdings sind die CCM-Strompulse nicht über die gesamte ventrikuläre Kontraktion verfügbar, so dass im Gegensatz zum HDS keine Bestimmung eines dem Schlagvolumen entsprechenden Parameters möglich ist, jedoch aber dafür eine Verlaufskontrolle des enddiastolische Volumens (EDV) als Steuerparameter für die CCM-Therapie verfügbar wird. (siehe Abbildungen 6, 7 und 8, 9).

Gemäß der letztgenannten Ausführungsvariante ist der Herzstimulator ein CRM-Stimulator (Schrittmacher, ICD, CRT-D) mit
- einem Closed Loop Sensor (CLS) zur Frequenzsteuerung und
- einem Modul zur kardialen Kontraktilitätsmodulation (CCM) und
- einer weiteren impedanzsensorischen Vorrichtung, die das Abbild der CCM-Konstantstrompulse auf die bipolare linksventrikuläre Elektrode wahrnimmt und diese in einen Verlaufsparameter des enddiastolischen Volumens umrechnet und
- mit zusätzlich mindestens einer Vorrichtung zur Koordination zwischen der CRM-Stimulation und CCM-Therapie,
wobei die CCM-Therapie dabei immer auch in Abhängigkeit des CLS-Sensorsignals gesteuert wird.

Die Wahrnehmung der Kontraktilitätsinformation, d.h. die Bestimmung des Herzkammervolumen-bezogenen Wertes mittels Impedanzmessung kann auch in durch die Closed-Loop-Stimulation an sich bekannter Manier mittels einer kontinuierlichen intrakardialen Impedanzmessung an einer ventrikulären Elektrode wahlweise im rechten Ventrikel, im linken Ventrikel, bipolar oder unipolar erfolgen.

Der Herzstimulator für die kardiale Kontraktilitätsmodulationstherapie ist vorzugsweisegleichzeitig auch ein implantierbarer Kardioverter/Defibrillator (ICD), ein biventrikulärer Herzschrittmacher für die kardiale Resynchronisationstherapie (CRT-P) oder eine Kombination aus einem biventrikulären Herzschrittmacher für die kardiale Resynchronisationstherapie und einem Kardioverter/Defibrillator (CRT-D).

Die Erfindung soll nun Anhand von Ausführungsbeispielen mit Bezug auf die Abbildungen näher erläutert werden. Von den Abbildungen zeigt:
- Fig. 1: einen implantierbaren Herzstimulator als CCM-CRT-D
- Fig. 2: ein Blockschaltbild einiger Komponenten des Herzstimulators aus Abb. 1;
- Fig. 3: Messpulse zur Kontraktilitätsmessung;
- Fig. 4: einen CLS-Sensor;
- Fig. 5: ein Beispiel einer physiologischen CCM-Aktivierung
- Fig. 6: HDS-Impedanzmessung
- Fig. 7: HDS-Impedanzmessung
- Fig. 8: CCM-Impedanzmessung
- Fig. 9: CCM-Impedanzmessung

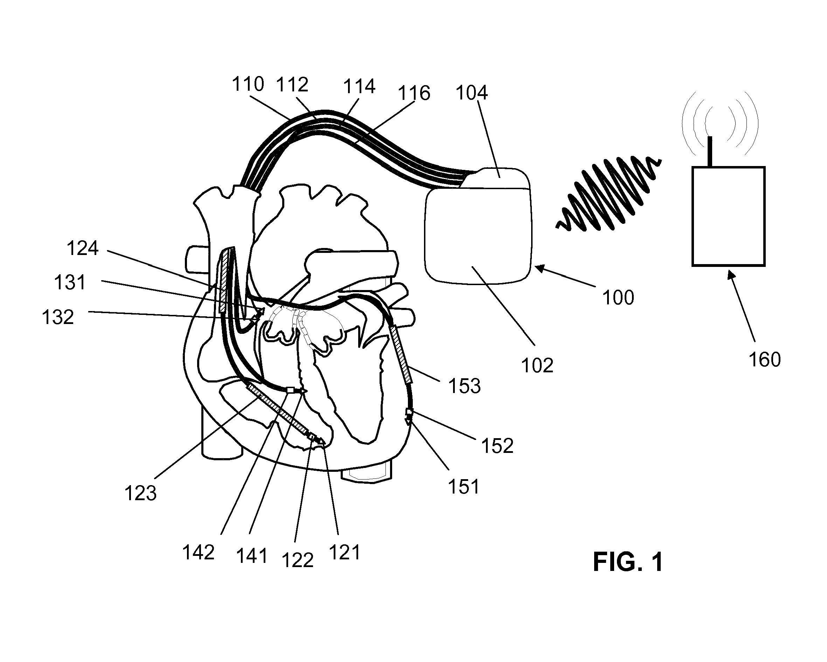

In der Fig. 1 ist als Implementierungsbeispiel ein Dreikammer-ICD-System mit umschaltbarer CRT-CCM-Funktion dargestellt. Der Herzstimulator (Impulsgenerator oder auch schlicht Generator) 100 ist mit mehreren implantierbaren Elektrodenleitungen 110, 112, 114 und 116 verbunden. Der Generator 100 besitzt ein Gehäuse 102, das im Beispielsfall aus Metall und daher elektrisch leitend sowie hermetisch dicht ist. In dem Gehäuse 102 sind elektronische und elektrische Komponenten des Generators 100 wie z.B. eine Batterie, Steuerelektronik, Stimulationsimpulsgeneratoren, Wahrnehmungseinheiten (auch als Sensing-Einheiten bezeichnet) mit entsprechender Verstärker- und Filterelektronik untergebracht. Diese Komponenten sind über elektrische Steckverbindungen in einem Anschlussgehäuse 104 (auch als Header bezeichnet) des Generators 100 mit den Elektrodenleitungen 110, 112, 114 und 116 lösbar verbunden.

Zur rechtsventrikulären Wahrnehmung und Stimulation umfasst die rechtsventrikuläre Elektrodenleitung 110 eine rechtsventrikuläre Tipelektrode 121 und eine rechtsventrikuläre Ringelektrode 122 am distalen Ende. Über die rechtsventrikuläre Tipelektrode 121 werden die Messpulse für den CLS-Sensor abgegeben. Die Gegenelektrode bildet hier das Generatorgehäuse 100. Zur Defibrillationsschockabgabe sind an dieser Elektrodenleitung eine distale 123 und optional auch eine proximale Schockwendel 124 angebracht.

Die rechtsatriale Elektrodenleitung 116 weist an ihrem distalen Ende einen bipolare Wahrnehmungs- und Stimulationspol (mit einer rechtsatrialen Tip-Elektrode 131 und einer rechtsatrialen Ring-Elektrode 132) auf und dient der Wahrnehmung des atrialen Rhythmus und bei Bedarf der atrialen Stimulation.

Zur CCM-Stimulation - also zur kardialen Kontraktilitätsmodulationstherapie - sind ein oder mehrere rechtsventrikuläre Septale-Elektrodenleitungen 112 vorgesehen, die über eine Tipelektrode 141 und Ringelektrode 142 die CCM-Pulse jeweils an das ventrikuläre Septum abgeben.

Optimal umfasst das System auch eine linksventrikuläre bzw. CS (Coronar Sinus) Elektrodenleitung zur Angabe von Stimulationsimpulsen zur kardialen Resynchronisation (CRT) über eine linksventrikuläre Tipelektrode 151 und eine linksventrikuläre Ringelektrode 152. Zur effektiveren Defibrillation/Kardioversion ist optional eine linksventrikuläre Schockwendel 153 vorgesehen.

Die Kommunikation mit externen Programmier- und Steuer- und Datenübertragungsgeräten 160 erfolgt über eine drahtlose, bidirektionale Telemetrieeinheit.

Der Herzstimulator 100 nutzt folgenden Zusammenhang:
Bei einem physiologischen Frequenzanstieg durch eine physische oder psychische Belastung wird neben der Herzfrequenz immer auch die Kontraktilität gesteigert (positive Inotropie). Dieser Zusammenhang wird bislang zur Frequenzsteuerung von Schrittmachersystemen genutzt, indem mittels einer rechtsventrikulären unipolaren Impedanzmessung die Kontraktionsdynamik des Herzens erfasst wird und diese Information zusammen mit einer Plausibilitätsprüfung des Akzelerometersensors genutzt wird, um bei chronotrop inkompetenten Patienten eine physiologische Closed Loop Frequenzsteuerung zu realisieren (Prinzip der Biotronik CLS-Schrittmacher). Die dabei erfasste, aus dem Impedanzsignal abgeleitete Sensorgröße ist nachweislich für die Frequenzsteuerung eines Schrittmachers geeignet, nicht jedoch als eine direkte Messgröße für die Haemodynamik zu verstehen *[*Kaye G, Arthur W, Edgar D, Lippert M, Czygan G. " The use of unipolar intracardiac impedance for discrimination of haemodynamically stable and unstable arrhythmias in man. " Europace 2006;8:988-93*].*

Für die physiologische Steuerung der CCM-Therapie wird nun das genannte CLS-Prinzip derart genutzt, dass ausgehend von einer chronotropen Kompetenz eine inotrope Inkompetenz durch den CLS-Sensor festgestellt wird und im Bedarfsfall eine CCM-Therapie eingeleitet wird.

Vorzugsweise wird immer dann eine CCM-Therapie abgegeben, wenn der durch den CLS-Sensor vorgegebene Frequenzanstieg dem tatsächlichen Herzfrequenzanstieg nachhängt. Vorausgesetz ist dabei ein Nachweis von Sinusrhythmus. Je nach Grunderkrankung und Ausprägung der Herzinsuffizienz sind auch andere Regelalgorithmen nach gleichem Grundprinzip realisierbar.

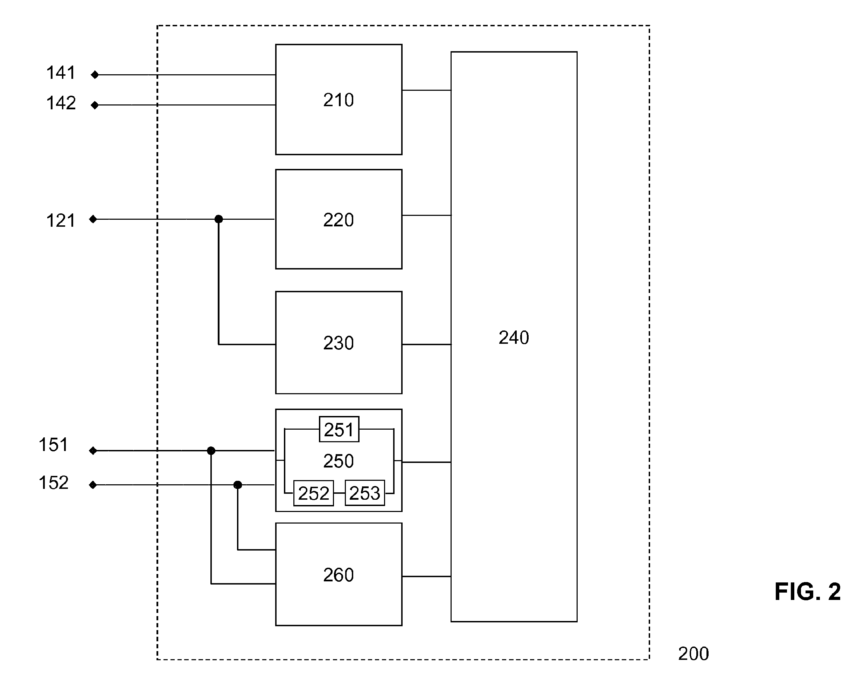

In der Fig. 2 ist schematisch vereinfachtes Blockschaltbild eines einfachen, CLS/HDSgesteuerten CCM-Systems dargestellt. Dieses umfasst hier auszugsweise folgende aus Abbildung 1 beschriebene Komponenten: Generator 100, rechtsventrikuläre Tip-Elektrode 121 zur unipolaren Impedanzmessung für den CLS-Sensor und die CCM-Elektrode 141, 142.

Die CCM-Elektrodenanschlüsse 141, 142 sind mit einem CCM-Pulsgenerator/Synchronisationseinheit 210 verbunden, die die typischen CCM-Impulse selbsttätig in die absolute ventrikuläre Refraktärzeit abgibt. Diese Pulse werden ebenfalls als HDS-Messpulse verwendet. Der CCM-Pulsgenerator ist eine Stimulationseinheit.

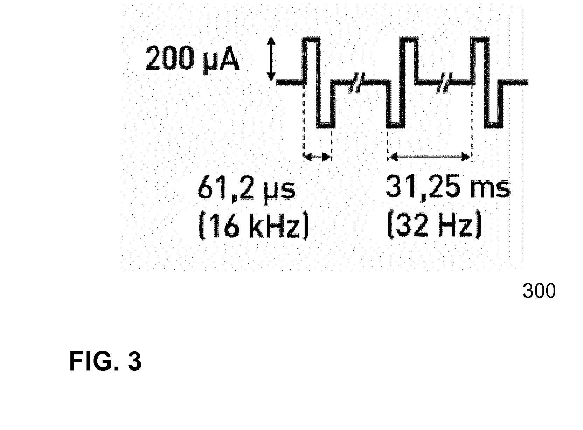

Der rechtsventrikuläre Elektrodenanschluss 121 ist mit einer Sensing- und Herzrhythmuserfassungseinheit 220 verbunden, um die ventrikuläre Rate und ggf. auch die ventrikuläre Refraktärzeit zu bestimmen. Zusätzlich ist die rechtsventrikuläre Elektrode 121 mit einer unipolaren Impedanzerfassungseinheit 230 zur Bestimmung des CLS-Signals verbunden. Diese Impedanzerfassungseinheit 230 gibt über die rechtsventrikuläre Elektrode 121 und das Generatorgehäuse 200 Konstantstrompulse zur Bestimmung der intrakardialen Impedanz während einer ventrikulären Kontraktion ab. Die Konstantstrompulse sind in Abbildung 3 dargestellt.

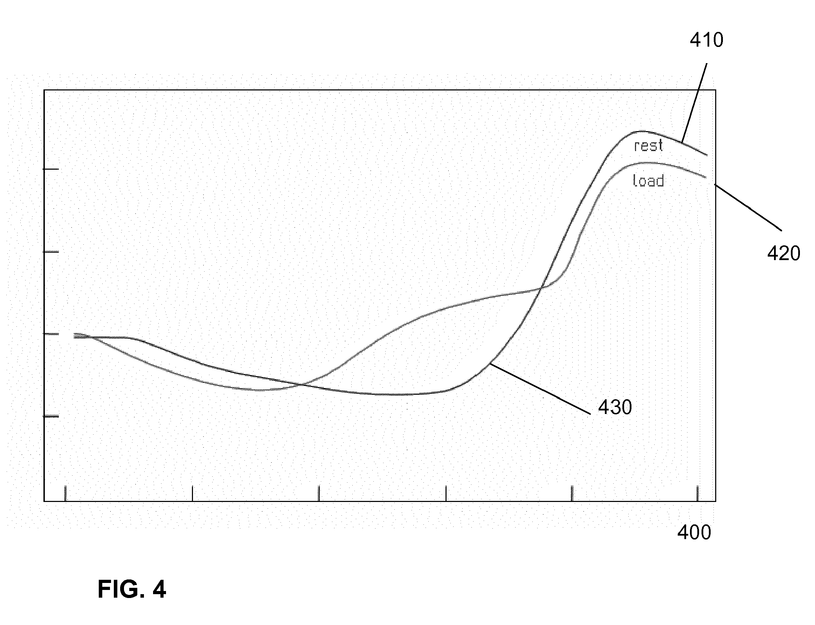

Herzrhythmuserfassungseinheit 220 und Impedanzmesseinheit 230 sind mit einer Auswerte- und Steuereinheit 240 verbunden. Diese wertet die Frequenz- und CLS-Signale derart aus, dass ein Beziehung zwischen Herzrate und CLS-Signal hergestellt wird und in Abhängigkeit dessen (siehe Abbildung 4) der mit der Auswerte- und Steuereinheit ebenfalls verbundene CCM-Pulsgenerator 210 aktiviert oder deaktiviert wird.

Die bipolaren Anschlüsse der linksventrikulären Elektroden 151, 152 sind mit der linksventrikulären Stimulations- und Wahrnehmungseinheit 250 und zusätzlich mit der Impedanzauswertungseinheit 260 verbunden. Diese Impedanzauswertungseinheit wertet die durch die CCM-Impulse generierten Spannungsänderungen an der bipolaren Elektrode 151, 152 derart aus, dass damit ein der enddiastolischen Füllung entsprechender Parameter bestimmt werden kann.

In der Fig. 3 sind die typischen Stromimpulse 300 zur kontinuierlichen intrakardialen Impedanzmessung mittels eines impedanzbasierten hämodynamischen Sensors (HDS-Sensors) dargestellt. Die Stromimpulse 300 werden z.B. kontinuierlich bzw. immer nach einem ventrikulären Ereignis für eine bestimmte Zeit, jeweils zwischen der rechtsventrikulären Tipelektrode 121und dem Generatorgehäuse 102 abgegeben. Die Aufzeichnung der benötigten Spannung für die Konstantstromeinspeisung ergibt dann das CLS-Rohsignal.

In der Fig. 4 ist die Bestimmung des CLS-Sensorsignals aus dem gemessenen unipolaren rechtsventrikulären Impedanzsignal vereinfacht dargestellt.

Im System wird kontinuierlich eine Referenzimpedanzkurve 410 unter Ruhebedingungen (z.B. bei entsprechend niedriger Herzfrequenz o.ä.) aufgenommen. Diese Referenzkurve wird kontinuierlich mit einer aktuellen Impedanzkurve 420 verglichen. Der Vergleich findet dabei vorzugsweise durch die Bestimmung der Flächendifferenz 430 beider Kurven statt. Überschreitet diese Flächendifferenz einen bestimmten Wert, so kann von einer gesteigerten Kontraktionsdynamik ausgegangen werden.

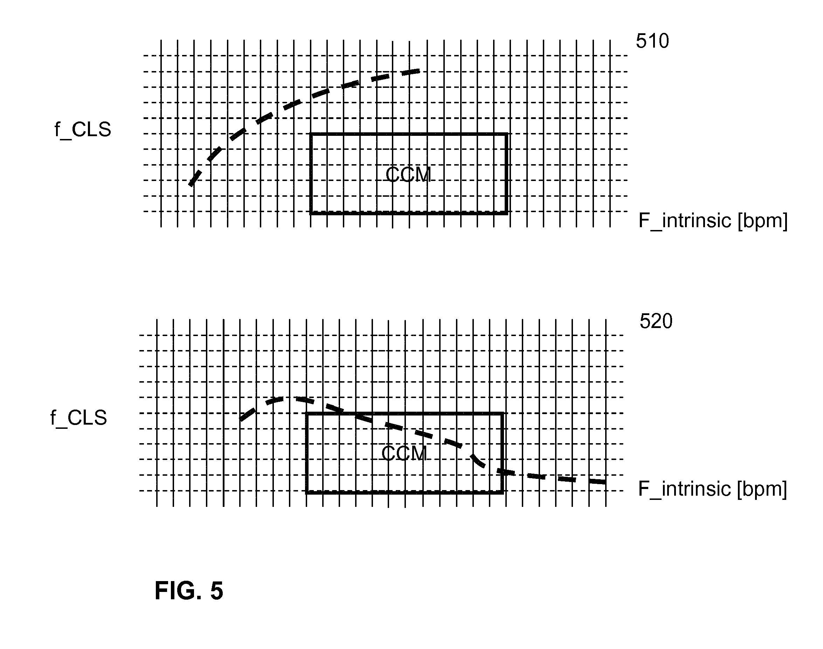

In der Fig. 5 ist nur eine mögliche Steuerung der kardialen Kontraktilitätsmodulationstherapie (CCM-Therapie) mit o.g. System illustriert. Folgt das CLS-Sensorsignal (f_CLS) in physiologisch hinreichendem Maß einer intrinsischen Herzfrequenzsteigung (f_intrinsic), so bleibt die CLS-Kurve außerhalb des CCM-Therapiebereiches 510. Ist die nicht der Fall, so wird eine CCM-Therapie aktiviert 520, sofern die Herzfrequenz nicht eine kritische Frequenz überschreitet.

Die Festlegung des therapiepflichtigen Bereiches dabei ist patientenindividuell programmierbar.

Das dargestellte Grundprinzip kann um eine Nachweisvorrichtung von Sinusrhythmus (mittels atrialer Signale) ergänzt werden, um die intrinsische Frequenzsteigerung nur bei physiologischem Frequenzanstieg und nicht bei pathophysiologischem Frequenzanstieg auszuwerten. Hier kann z.B. ein bekannter Diskriminierungsalgorithmus wie Biotronik SMART eingesetzt werden.

In der Fig. 6 ist das als HDS-Prinzip bekannte Messverfahren zur Bestimmung des relativen Schlagvolumens dargestellt. Hier wird über die bipolare rechtventrikuläre Elektrode 610 über den gesamten Herzzyklus ein Konstantstrom eingeprägt und an der bipolaren linksventrikulären Elektrode 620 wird dessen Abbild wahrgenommen.

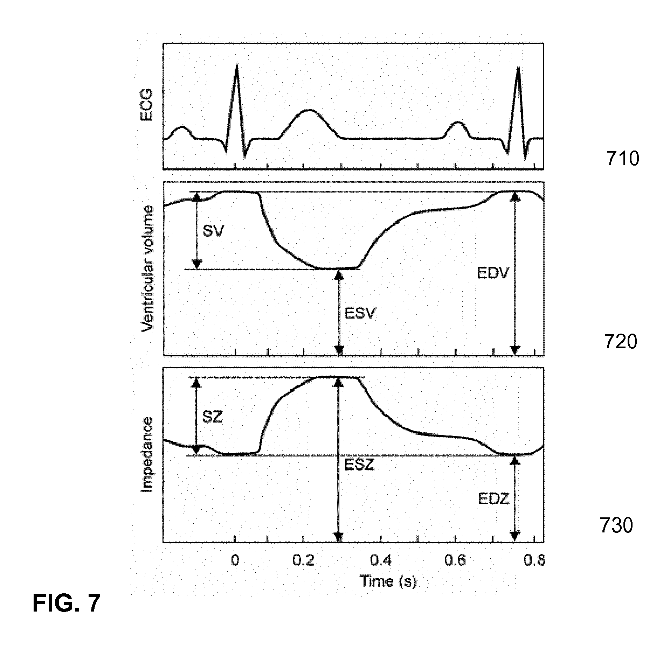

Die Fig. 7 zeigt nun die Bestimmung des Schlagvolumens (SV) aus der HDS-Impedanzkurve 730. Im oberen Kanal ist das Oberflächen-EKG 710 dargestellt und darunter der Verlauf der ventrikulären Volumenkurve 720. Die mit dem HDS-Prinzip aufgezeichnete Impedanzkurve 730 verhält sich invers zur Volumenkurve 720, da sich ein großes ventrikuläres Blutvolumen in einer reduzierten Impedanz auf Grund der guten Leitfähigkeit von Blut niederschlägt. Aus der Differenz (SZ) der enddiastolischen (EDZ) und endsystolischen Impedanz (ESZ) kann nun ein Parameter gewonnen werden, der zum Schlagvolumen (SV) korreliert, d.h. der relative Verlauf des Schlagvolumens kann so aufgezeichnet und für die Implantatssteuerung genutzt werden.

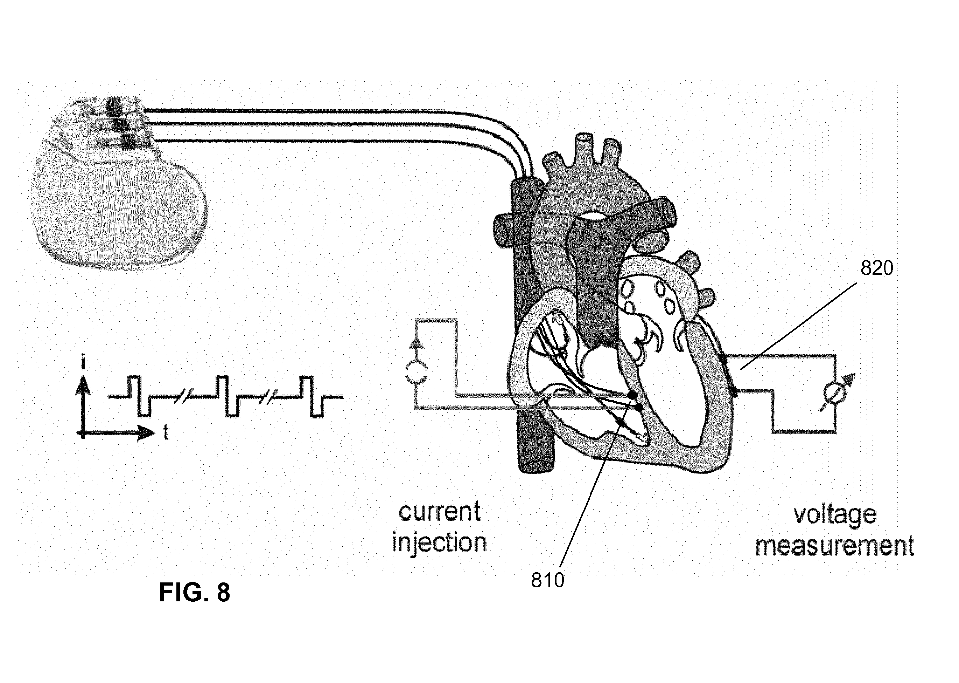

In der Fig. 8 ist nun das an das HDS-Prinzip angelehnte Messverfahren zur CCM-Impedanzmessung zur Bestimmung des relativen enddiastolischen Volumens dargestellt. Hier erfolgt die Stromeinspeisung über die bipolaren septalen CCM-Elektroden 810 in Form der CCM-Therapieimpulse.

Die bipolare linksventrikuläre Elektrode 820 wird wiederum zur Wahrnehmung der Impedanztestpulse verwendet.

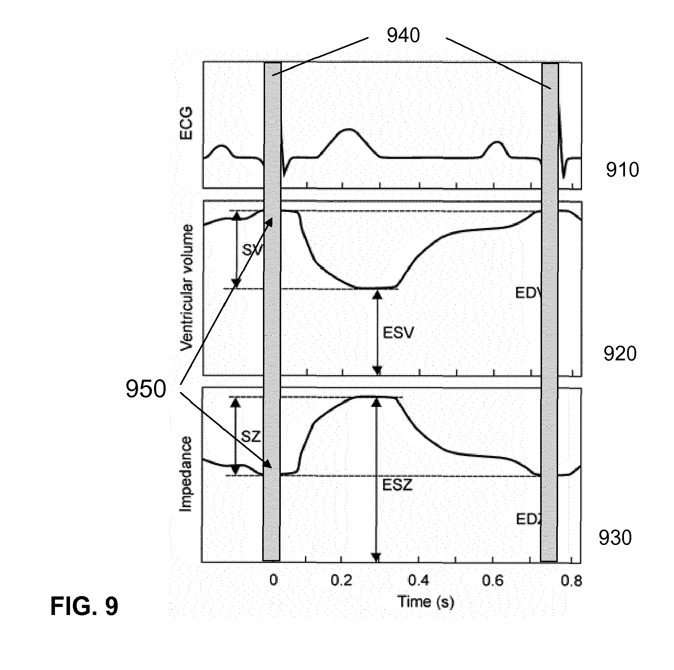

In der Fig. 9 wird die Bestimmung des enddiastolischen Volumens gezeigt (910: EKG, 920: Ventrikelvolumen, 930: Impedanzkurve). Die CCM-Impulse sind hinsichtlich der Stromamplitude deutlich größer als die zulässigen HDS-Messpulse. Daher können diese CCM-Pulse nur während der absoluten Refraktärzeit und nicht über den gesamten Herzzyklus abgegeben werden, ohne eine Arrhythmie zu induzieren. Aus diesem Grund kann die Impedanz-Messung mittels der CCM-Impulse nur in den definierten Messfenstern 950 für einige 10 Millisekunden ausgeführt werden und erlaubt so keine Bestimmung eines Schlagvolumenparameters. Allerdings können die Messungen der einzelnen Messfenster einen relativen Verlauf des enddiastolischen Volumens aufzeichnen und damit als geeigneter Parameter für die Implantatsteuerung, insb. auch der CCM-Therapie, genutzt werden.

Vorteile der Nutzung der CCM-Impulse sind die zu erwartenden wesentlich besseren Signal-Rauschverhältnisse der Messung und die günstige Lage der septalen Elektrode zur Stromeinspeisung.

Der vorgestellte Herzstimulator bietet den Vorteil, eine potentielle Dekompensation durch eine dauerhafte CCM-Stimulation zu vermeiden und gleichzeitig den für die CCM-Stimulation benötigten Energiebedarf signifikant zu senken.

Die Lösung hat dabei den entscheidenden Vorteil, dass der für die physiologische CCM-Steuerung eingesetzte CLS-Sensor bereits als Sensor für die Frequenzanpassung operieren kann.

Zusätzlich bietet die Bestimmung des enddiastolischen Volumens unter CCM-Therapie eine adäquate Verfolgung des Therapieverlaufes und kann zur Implantatsteuerung ohne zusätzlich erforderliche Elektroden eingesetzt werden.

## Patentansprüche

1. Herzstimulator
- mit wenigstens einer Stimulationseinheit, die mit einer oder mehreren Stimulationselektroden verbunden oder zu verbinden und ausgebildet ist, zumindest unterschwellige Stimulationsimpulse für eine kardiale Kontraktionsmodulationstherapie abzugeben,
- mit einer Impedanzerfassungseinheit, die mit einer oder mehreren Elektroden verbunden oder zu verbinden und ausgebildet ist, eine infolge eines jeweiligen unterschwelligen Strom- oder Spannungsimpulses auftretende Spannung oder Stromstärke zu erfassen und daraus einen jeweiligen Impedanzwert abzuleiten,
- einer Impedanzauswertungseinheit, die ausgebildet ist, wenigstens einen Herzkammervolumen-bezogenen Wert und/oder einen Atemzeitvolumen-bezogenen Wert und/oder einen Kontraktionszeit-bezogenen Wert zu bestimmen und
- mit einer Steuereinheit, die mit der Stimulationseinheit und der Herzrhythmuserfassungseinheit verbunden und ausgebildet ist, eine Stimulationsimpulsabgabe über die Stimulationseinheit derart zu steuern, dass der Herzstimulator unterschwellige Stimulationsimpulse für eine kardiale Kontraktionsmodulationstherapie abgeben kann
**dadurch gekennzeichnet, dass** die Steuereinheit mit der Impedanzauswertungseinheit verbunden und ausgebildet ist, eine Abgabe unterschwelliger Stimulationsimpulse für eine kardiale Kontraktionsmodulationstherapie in Abhängigkeit des Herzkammervolumen-bezogenen Wertes und/oder des Atemzeitvolumen-bezogenen Wertes und/oder des Kontraktionszeit-bezogenen Wertes zu steuern.

2. Herzstimulator nach Anspruch 1, **dadurch gekennzeichnet, dass** ein jeweiliger für eine Impedanzmessung genutzter unterschwelliger Strom- oder Spannungsimpulse ein Stimulationsimpuls für die Kontraktionsmodulationstherapie ist.

3. Herzstimulator nach Anspruch 1 oder 2, **dadurch gekennzeichnet, dass** der Herzstimulator eine Herzrhythmuserfassungseinheit aufweist, die ausgebildet ist eine intrinsische Herzfrequenz zu erfassen, wobei die Steuereinheit mit der Herzrhythmuserfassungseinheit verbunden und ausgebildet ist, aus dem Herzkammervolumen-bezogenen Wert eine indizierte Herzfrequenz abzuleiten und eine Abgabe unterschwelliger Stimulationsimpulse für eine kardiale Kontraktionsmodulationstherapie zu unterbinden, wenn die durch den Herzkammervolumen-bezogenen Wert indizierte Herzfrequenz oder eine Änderung der indizierten Herzfrequenz einer intrinsischen Herzfrequenz bzw. der Änderung einer intrinsischen Herzfrequenz qualitativ entspricht.

4. Herzstimulator nach einem der Ansprüche 1 bis 3, bei dem die Impedanzauswertungseinheit Teil eines CLS-Sensors oder HDS-Sensors zum Generieren eines Steuersignals für eine Stimulationsratensteuerung auf Basis Herzkammervolumen-basierter Werte ist.

5. Herzstimulator nach einem der Ansprüche 1 bis 4, bei dem die Impedanzauswertungseinheit Teil einer Kombination eines CLS- und Atemminutenvolumensensors ist.

6. Herzstimulator gemäß einem der Ansprüche 1 bis 5, **dadurch gekennzeichnet, dass** die Steuereinheit ausgebildet, eine kardiale Kontraktionsmodulationstherapie immer dann einzuleiten, wenn die Impedanzauswertungseinheit eine Herzfrequenzsteigerung vorgibt.

7. Herzstimulator gemäß einem der Ansprüche 1 bis 5, **dadurch gekennzeichnet, dass** die Steuereinheit ausgebildet, eine kardiale Kontraktionsmodulationstherapie immer dann einzuleiten, wenn die Impedanzauswertungseinheit eine Reduktion der Herzfrequenz vorgibt.

8. Herzstimulator nach Anspruch 6, **dadurch gekennzeichnet, dass** die Steuereinheit ausgebildet ist, eine kardiale Kontraktionsmodulationstherapie immer dann zu starten, wenn die intrinsische Herzfrequenz des Patienten, bei nachgewiesenem Sinusrhythmus ansteigt, ein von der Impedanzauswertungseinheit vorgegebener Stimulationsratenanstieg jedoch hinter dem intrinsischen Herzfrequenzanstieg zurückbleibt

9. Herzstimulator gemäß einem der Ansprüche 1 bis 8, **dadurch gekennzeichnet, dass** der Herzstimulator zusätzlich einen Aktivitätssensor aufweist, der ebenfalls ein Ausgangssignal liefert, dass einen Hinweis auf den metabolischen Bedarf eines Patienten gibt.

10. Herzstimulator gemäß Anspruch 9, **dadurch gekennzeichnet, dass** die Steuereinheit ausgebildet ist, ein Ausgangssignal des Aktivitätssensors zur Plausibilitätskontrolle zu verarbeiten.

11. Herzstimulator gemäß Anspruch 9 oder 10, **dadurch gekennzeichnet, dass** die Steuereinheit ausgebildet ist, eine kardiale Kontraktionsmodulationstherapie immer dann einzuleiten, wenn das Ausgangssignal der Impedanzauswertungseinheit eine Herzfrequenzsteigerung indiziert, das Ausgangssignal des Aktivitätssensors hingegen keine Frequenzsteigerung anzeigt.

12. Herzstimulator gemäß einem der Ansprüche 1 bis 11, **dadurch gekennzeichnet, dass** der Herzstimulator ausgebildet ist, Herzkammervolumen-bezogene Werte mittels Impedanzmessung mittels einer kontinuierlichen intrakardialen Impedanzmessung an einer ventrikulären Elektrode zu bestimmen.

13. Herzstimulator gemäß einem der Ansprüche 1 bis 12, **dadurch gekennzeichnet, dass** der Herzstimulator ein Herzstimulator für die kardiale Kontraktilitätsmodulationstherapie ist und gleichzeitig auch ein implantierbarer Kardioverter/Defibrillator (ICD), ein biventrikulärer Herzschrittmacher für die kardiale Resynchronisationstherapie (CRT-P) oder eine Kombination aus einem biventrikulären Herzschrittmacher für die kardiale Resynchronisationstherapie und einem Kardioverter/Defibrillator (CRT-D).
